# EUROPÄISCHE PATENTANMELDUNG

(11) **EP 0 825 182 A1**
(43) Veröffentlichungstag der Anmeldung: **25.02.1998**
(21) Anmeldenummer: 97114469.6
(22) Anmeldetag: 21.08.1997
(51) Int. Cl.: C07D 211/74

(54) **Verfahren zur Herstellung von 2,2,6,6-Tetramethylpiperidin-4-on (TAA)**

(30) Priorität: 23.08.1996 DE 19634157
(71) Anmelder: BASF AKTIENGESELLSCHAFT, 67056 Ludwigshafen (DE)
(72) Erfinder: Julius, Manfred, Dr., 67117 Limburgerhof (DE); Hermeling, Dieter, Dr., 67459 Böhl-Iggelheim (DE); Siegel, Hardo, Dr., 67346 Speyer (DE); Harder, Wolfgang, Dr., 69469 Weinheim (DE)
(74) Vertreter: Isenbruck, Günter, Dr.

(57) **Zusammenfassung**

Es wird ein Verfahren zur Herstellung von 2,2,6,6-Tetramethylpiperidin-4-on (TAA) beschrieben, bei dem
- eine Mischung, enthaltend Aceton und/oder Aceton-Kondensate und Ammoniak in einem Molverhältnis von 20:1 bis 3:1
- bei einer Temperatur von 50°C bis 130°C
- in Gegenwart von Dimethylsulfat zur Reaktion gebracht wird, wobei die Menge des Dimethylsulfats 0,5 bis 5 Mol-%, bezogen auf Aceton, beträgt.

## Beschreibung

Die Erfindung betrifft ein Verfahren zur Herstellung von 2,2,6,6-Tetramethylpiperidin-4-on (TAA).

Triacetonamin (2,2,6,6-Tetramethylpiperidin-4-on; TAA) kann durch Umsetzung von Aceton oder von acetonhaltigen Lösungen von Aceton-Folgeprodukten bzw. -Kondensaten, wie etwa Mesityloxid, Diacetonalkohol, Diacetonamin, Acetonin oder Phoron mit Ammoniak in einer Cyclokondensationreaktion hergestellt werden.

Die Synthese kann ein- oder zweistufig durchgeführt werden. Das zweistufige Verfahren ist kostenungünstiger. Dabei verläuft die Synthese des TAA über intermediär gebildetes Acetonin als Zwischenstufe.

Die kostengünstigere einstufige Synthese kann sowohl homogen- als auch heterogenkatalysiert erfolgen. Die dabei eingesetzten Reaktionstemperaturen liegen üblicherweise im Bereich von 50 bis 100°C.

Aus der EP-B 0 004 104 ist ein Verfahren zur Herstellung von TAA bekannt, bei dem Aceton und Ammoniak in einem Molverhältnis von 2:1 bis 25:1 in Gegenwart eines festen, sauren Katalysators bei einer Temperatur in einem Bereich von 80°C bis 130°C beim Eigendruck des Systems, der auch durch Inertgaszugabe erhöht werden kann, umgesetzt werden. Die bei diesem bekannten Verfahren eingesetzten festen, sauren Katalysatoren sind sowohl in den Ausgangsstoffen als auch im Reaktionsgemisch praktisch unlöslich. Derartig feste Katalysatoren bieten den Vorteil, daß ihre chemische Beständigkeit sehr groß ist. Außerdem kann dieses bekannte Verfahren kontinuierlich durchgeführt werden. Nichtsdestotrotz ist das Verfahren insofern nachteilig, als die dabei erzielten Ausbeuten gering sind. Sie liegen etwa bei 19 %, bezogen auf eingesetztes Aceton.

Andere bekannte TAA-Syntheseverfahren erfolgen unter diskontinuierlichen Bedingungen, was einen erhöhten Arbeits- und Zeitaufwand zur Folge hat (vgl. z.B. DE-C 29 10 761).

Bei den diskontinuierlichen Verfahren sind unterschiedliche Katalysatoren eingesetzt worden, z.B. Lewis-Säuren, Brönstedt-Säuren, Halogenverbindungen u.a. Allen diesen Verfahren ist gemeinsam, daß der Katalysator nach Durchführung der Reaktion unter großem Aufwand abgetrennt und beseitigt werden muß. Wenn beispielsweise Calciumchlorid verwendet wird (vgl. ES-A 479 049), so fallen bei der Reaktion größere Mengen an wäßriger Calciumchloridlösung an, die wegen ihrer Verunreingung mit organischen Substanzen nur schwierig zu beseitigen sind.

Ähnliche Schwierigkeiten ergeben sich auch bei der Synthese des TAA unter Verwendung von Katalysatoren, die zu den Halogenverbindungen gehören. Die dabei entstehenden Abfallstoffe sind vor allem unter ökologischen Gesichtspunkten problematisch.

Aufgabe der Erfindung ist es daher, ein Verfahren - insbesondere eines der kontinuierlichen Art - bereitzustellen, bei dem das Syntheseprodukt TAA in großer Ausbeute erhalten wird und bei dem zugleich der Zeit- und Arbeitsaufwand gering ist. Außerdem soll es in Hinsicht auf die vorstehend genannten Probleme in ökologischer Hinsicht weniger problematisch sein.

Diese Aufgabe wird durch ein Verfahren zur Herstellung von TAA gelöst, umfassend die Schritte, daß eine Mischung, enthaltend Aceton und/oder Aceton-Kondensate und Ammoniak in einem Molverhältnis von 20:1 bis 3:1 bei einer Temperatur von 50°C bis 130°C in Gegenwart von Dimethylsulfat zur Reaktion gebracht wird, wobei die Menge des Dimethylsulfats, bezogen auf Aceton, 0,5 bis 5 Mol-% beträgt.

Bevorzugte Ausführungsformen sind Gegenstand der Unteransprüche.

Kernpunkt der Erfindung ist, daß Dimethylsulfat (H₃C-OSO₃-CH₃;DMS) einen ausgezeichneten Katalysator für die Synthese von TAA aus Aceton und Ammoniak darstellt. Dieser Katalysator erfüllt alle vorstehend erwähnten Anforderungen gleichzeitig. Er ist in Aceton und Ammoniak gut löslich, d.h. also auch gut dosierbar. Außerdem ist der Katalysator kostengünstig. Hinzu kommt, daß es bei Durchführung der Synthese unter Einsatz dieses Katalysators zu homogenen, halogenfreien Reaktionsausträgen kommt, was insbesondere unter ökologischen Gesichtspunkten ein deutlicher Vorteil ist.

Bei dem erfindungsgemäßen Verfahren wird das Dimethylsulfat in einer Menge von 0,5 bis 5 Mol-%, bezogen auf Aceton, eingesetzt. Das Molverhältnis Aceton zu Ammoniak im Reaktionsansatz beträgt 20:1 bis 3:1, bevorzugt jedoch 10:1 bis 3:1. Besonders gute Selektivitäten der Umsetzung weden bei einem Molverhältnis von Aceton zu Ammoniak im Bereich von 10:1 bis 6:1 erzielt. Falls die Reaktionsmischung Aceton-Kondensate enthält, so werden diese auf Aceton umgerechnet; beispielsweise entspricht 1 Mol Mesityloxid 2 Mol Aceton.

Desweiteren eignet sich das erfindungsgemäße Verfahren auch hervorragend für die kontinuierliche Synthese. Dabei wird der Druck derart eingestellt, daß unter den Reaktionsbedingungen alle Komponenten flüssig vorliegen; bei kontinuierlicher Fahrweise in einem Temperaturbereich von 50°C bis 100°C liegt der Druck dann in einem Bereich von 10-100 bar [1x10⁶ - 1x10⁷ Pa]. Außerdem kann bei dem erfindungsgemäßen Verfahren nicht nur Aceton als Ausgangsstoff eingesetzt werden. Vielmehr können auch Folgeprodukte des Acetons, insbesondere durch Kondensationsreaktion erhaltene Folgeprodukte wie Mesityloxid, Diacetonalkohol, Diacetonamin, Acetonin oder Phoron alleine oder als Mischung in Kombination mit Aceton eingesetzt werden.

Die Erfindung wird nachfolgend durch Beispiele näher erläutert:

### I. Diskontinuierliche Synthese von TAA

### Beispiel 1:

In einem 3,5 l Autoklaven aus nichtrostendem Suthl wurden 30,2 Mol Aceton und 0,60 Mol Dimethylsulfat vorgelegt. Nach dem Inertisieren des Autoklaven mit Stickstoff wurden 4,5 Mol Ammoniak aufgepreßt und unter intensivem Rühren die Reaktionsmischung auf 60°C erwärmt. Es stellte sich ein Druck von ca. 5 bar ein.

Nach einer Reaktionszeit von 4 h kühlte man den Autoklaven auf Raumtemperatur ab, entspannte und analysierte den Reaktionsaustrag gaschromatographisch.

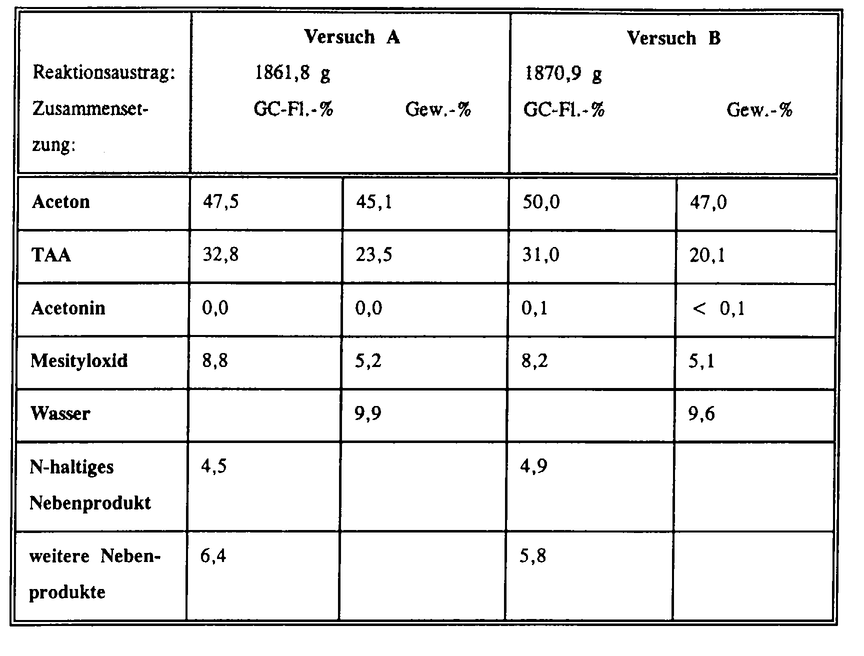

Hieraus ergibt sich im Mittel eine TAA-Ausbeute von 26,0 %, bezogen auf eingesetztes Aceton, bei einem Aceton-Umsatz von 51,0 %

### Beispiel 2:

Die analoge Umsetzung von 30,2 Mol Aceton entsprechend dem Beispiel Nr. 1, jedoch mit einer veränderten Menge an Ammoniak von 3,0 Mol ergab nach gaschromatographischer Analyse des Reaktionsaustrags folgendes Ergebnis:

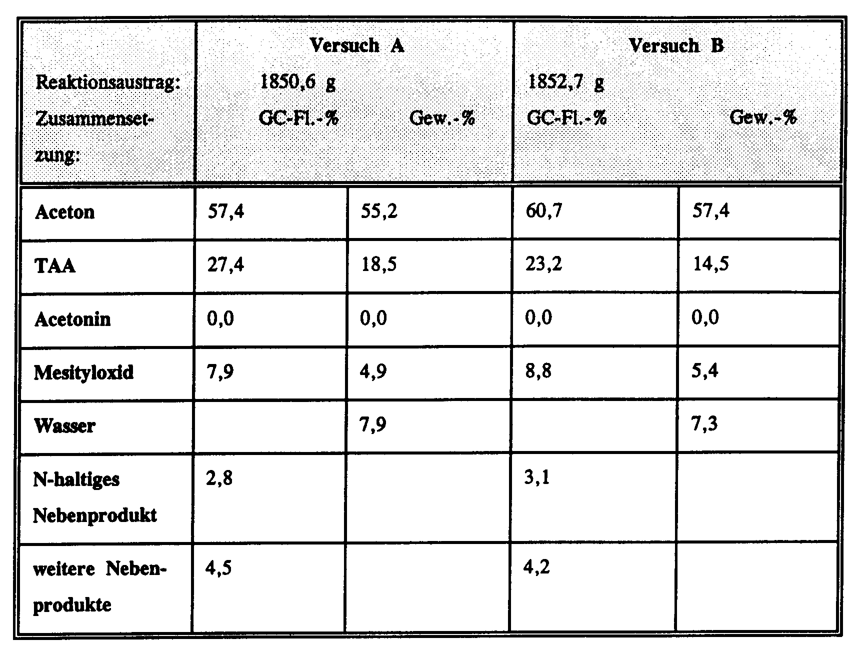

Hieraus ergibt sich im Mittel eine TAA-Ausbeute von 19,6 %, bezogen auf eingesetztes Aceton, bei einem Aceton-Umsatz von 40,6 %.

### Kontinuierliche Synthese von TAA

### Beispiel 3

Am Boden eines druckbeständigen Rührkessel-Reaktors mit einem Volumen von 184 cm³ wurde ein aus den beiden Teilströmen von 19,8 g/h einer Lösung mit 2,0 Mol-% Dimethylsulfat in Aceton und 1,25 g/h Ammoniak vorgemischter Zufuhrstrom eingeführt, während am Kopf des Reaktors der Reaktionsaustrag kontinuierlich entnommen wurde. Der Reaktor wurde in gefluteter Fahrweise betrieben, der Druck betrug 14 bar. Der Inhalt des Reaktors wurde mittels eines Scheibenrührers intensiv durchmischt und der Reaktor in einem Ölbad erhitzt, so daß das Reaktionsgemisch eine Temperatur von 60°C besaß. Den Reaktorablauf kontrollierte man in einer nachgeschalteten Sichtzelle (Volumen 5 ml) unter Reaktionsbedingungen bezüglich-Druck und Temperatur auf seine Einphasigkeit. Der homogene Reaktionsaustrag wurde druckgeregelt in einem Phasenscheider entspannt, gesammelt und gaschromatographisch analysiert.

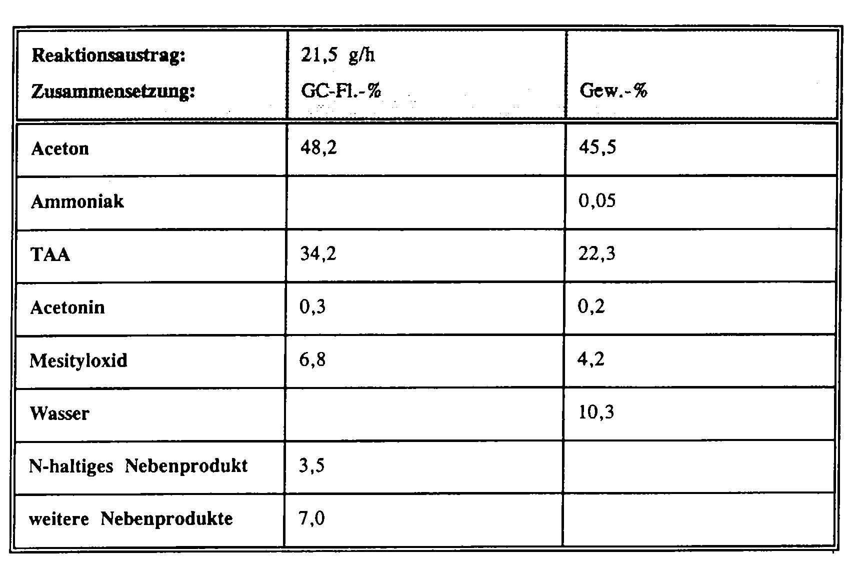

Hieraus ergibt sich eine TAA-Ausbeute von 27,9 %, bezogen auf eingesetztes Aceton, bei einem Aceton-Umsatz von 49,5 %.

## Patentansprüche

1. Verfahren zur Herstellung von 2,2,6,6-Tetramethylpiperidin-4-on (TAA), umfassend die Schritte, daß
- eine Mischung, enthaltend Aceton und/oder Aceton-Kondensate und Ammoniak in einem Molverhältnis von 20:1 bis 3:1
- bei einer Temperatur von 50°C bis 130°C
- in Gegenwart von Dimethylsulfat zur Reaktion gebracht wird, wobei die Menge des Dimethylsulfats 0,5 bis 5 Mol-%, bezogen auf Aceton, beträgt.

2. Verfahren nach Anspruch 1, dadurch gekennzeichnet, daß das Molverhältnis von Aceton und/oder Aceton-Kondensaten zu Ammoniak 10:1 bis 3:1 beträgt.

3. Verfahren nach Anspruch 1 oder 2, dadurch gekennzeichnet, daß die Reaktion kontinuierlich erfolgt.

4. Verfahren nach einem der Ansprüche 1 bis 3, dadurch gekennzeichnet, daß das Reaktionsgemisch in flüssiger Form vorliegt.

5. Verfahren nach einem der Ansprüche 1 bis 4, dadurch gekennzeichnet, daß es sich bei den Aceton-Kondensaten um Mesityloxid, Diacetonalkohol, Diacetonamin, Acetonin oder Phoron handelt.
